(19) **Europäisches Patentamt / European Patent Office / Office européen des brevets**

(11)  **EP 2 582 652 B1**

(12) **FASCICULE DE BREVET EUROPEEN**

(45) Date de publication et mention
de la délivrance du brevet:
**31.10.2018 Bulletin 2018/44**

(21) Numéro de dépôt: **11736123.8**

(22) Date de dépôt: **16.06.2011**

(51) Int Cl.:
*C07C 45/52* (2006.01)     *C07C 47/22* (2006.01)
*C07C 319/18* (2006.01)     *C07C 323/22* (2006.01)
*C07C 319/20* (2006.01)     *C07C 323/52* (2006.01)
*C07C 323/58* (2006.01)     *C07C 323/60* (2006.01)
*B01J 21/06* (2006.01)     *B01J 21/08* (2006.01)
*B01J 23/10* (2006.01)     *B01J 23/20* (2006.01)
*B01J 23/30* (2006.01)     *B01J 23/34* (2006.01)

(86) Numéro de dépôt international:
**PCT/FR2011/051375**

(87) Numéro de publication internationale:
**WO 2011/157959 (22.12.2011 Gazette 2011/51)**

(54) **PROCEDE DE PREPARATION D'ACROLEINE A PARTIR DE GLYCEROL OU DE GLYCERINE**

VERFAHREN ZUR HERSTELLUNG VON ACROLEIN AUS GLYCEROL ODER GLYCERIN

PROCESS FOR PREPARING ACROLEIN FROM GLYCEROL OR GLYCERIN

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **17.06.2010 FR 1054794**

(43) Date de publication de la demande:
**24.04.2013 Bulletin 2013/17**

(73) Titulaires:
• **Adisseo France S.A.S.**
  **92160 Antony (FR)**
• **Centre National de la Recherche Scientifique**
  **75794 Paris Cedex 16 (FR)**

(72) Inventeurs:
• **LAURIOL-GARBEY, Pascaline**
  **F-69009 Lyon (FR)**

• **BELLIERE-BACA, Virginie**
  **F-78570 Andresy (FR)**
• **LORIDANT, Stéphane**
  **F-69330 Meyzieu (FR)**
• **MILLET, Jean-Marc**
  **F-69006 Lyon (FR)**

(74) Mandataire: **Delorme, Nicolas et al
Cabinet Germain & Maureau
BP 6153
69466 Lyon Cedex 06 (FR)**

(56) Documents cités:
**FR-A1- 2 907 445     FR-A1- 2 920 767
FR-A1- 2 938 535     US-A1- 2008 214 384**

EP 2 582 652 B1

**Description**

La présente invention concerne un procédé catalytique de fabrication d'acroléine par déshydratation du glycérol ou glycérine et les applications d'un tel procédé.

On entend par glycérol, un glycérol purifié ou non, de préférence issu de la biomasse, et notamment un glycérol hautement purifié ou partiellement purifié. Un glycérol purifié possède une pureté supérieure ou égale à 98%, obtenu par distillation de glycérine. Un glycérol non purifié ou seulement partiellement purifié pourra être en solution dans du méthanol lorsqu'il provient par exemple d'une transestérification de triglycérides, comme décrit ci-après. On entend par glycérine, notamment, une glycérine d'origine naturelle, issue de l'hydrolyse d'huiles végétales et/ou de graisses animales, ou une glycérine d'origine synthétique, issue du pétrole, plus ou moins purifiée ou raffinée, ou bien brute. A titre d'exemple, une glycérine brute a un titre compris entre 80 et 85%. Ainsi, dans la suite de la description, on se réfère principalement à la conversion d'un glycérol ou d'une glycérine issue de la biomasse, mais l'invention n'y est bien entendu pas limitée et son intérêt s'étend à tous glycérols et glycérines, quels que soient leurs origines et leurs degrés de pureté.

L'épuisement progressif des énergies fossiles conduit les industriels à envisager l'utilisation de matières premières renouvelables issues de la biomasse pour la production de carburants. Dans ce contexte, le biodiesel est un carburant produit à partir d'huile végétale ou animale.

Ce produit jouit d'une aura verte en raison d'un bilan $CO_2$ nettement favorable par rapport aux énergies fossiles. Le diester® (ou EMVH, Esters Méthyliques d'Huiles Végétales) est un biodiesel fabriqué par transestérification des triglycérides présents dans les liquides oléagineux, notamment les huiles végétales de palme, colza et tournesol, par du méthanol. Cette transestérification coproduit approximativement, et suivant les procédés envisagés, 100 kg de glycérol par tonne de diester®. La partie non lipidique de la matière première utilisée, les tourteaux, est principalement mise à profit dans l'alimentation animale.

Ce biodiesel est utilisé en mélange dans le gazole. Les directives européennes 2001/77/EC et 2003/30/EC, qui seront appliquées dans un futur proche, projettent d'introduire 7% en 2010 et 10% à l'horizon 2015 de diester® dans les gazoles. Cette augmentation substantielle de la quantité de biodiesel produit va générer des quantités importantes de glycérol équivalentes à plusieurs centaines de milliers de tonnes/an.

Quelques 1500 utilisations du glycérol sont déjà répertoriées, parmi lesquelles les suivantes illustrent à titre d'exemples sa présence dans de nombreuses et diverses formulations :

- hydratants en pharmacie (dans les suppositoires et les sirops) ou en cosmétologie dans les crèmes hydratantes, les savons à la glycérine, les dentifrices,
- solvants dans l'industrie alimentaire,
- plastifiants ou lubrifiants dans l'industrie chimique.

Ces applications s'avéreront nettement insuffisantes pour absorber les quantités de glycérol qui seront coproduites avec les biodiesels et bien qu'en progression, le marché conventionnel du glycérol (savons, pharmacie, ...) ne pourra pas non plus absorber un tel surplus. Il est donc vital de trouver de nouvelles applications permettant de valoriser de très gros volumes de glycérol.

Devant ce constat, de nombreux débouchés ont été étudiés ces dernières années (voir M. Pagliaro et al., Angew. Chem. Int. Ed. (2007) 46, 4434 - 4440 ainsi que M. Pagliaro, M. Rossi: The Future of Glycerol, RSC Publishing, Cambridge (2008)), avec, en particulier, les six voies de valorisation suivantes :

- conversion en 1,3-propanediol et en 1,2-propanediol, notamment utilisés comme monomères de base dans la synthèse des polyesters et polyuréthanes,
- conversion en monoesters pour la chimie des lubrifiants,
- conversion en polyglycérols employés en tant qu'agents émulsifiants, additifs alimentaires,
- conversion en acroléine (par déshydratation) et acide acrylique (par déshydratation et oxydation),
- valorisation directe en tant qu'additifs pour l'alimentation animale.

L'acroléine et l'acide acrylique sont traditionnellement produits par oxydation ménagée en phase gazeuse du propylène par l'oxygène de l'air en présence de catalyseurs à base d'oxydes de molybdène et/ou bismuth. L'acroléine ainsi obtenue peut soit être directement intégrée dans un procédé en deux étapes de fabrication d'acide acrylique, soit être utilisée comme intermédiaire de synthèse. La production de ces deux monomères est donc étroitement liée au propylène q u i est, en substance, fabriqué par vapocraquage ou craquage catalytique de coupes pétrolières.

Les marchés de l'acroléine, l'un des plus simples des aldéhydes insaturés, et de l'acide acrylique sont gigantesques car ces monomères entrent dans la composition de nombreux produits de masse.

Par ailleurs, l'acroléine, composé hautement réactif de par sa structure, trouve de nombreuses applications, notamment comme intermédiaire de synthèse. Elle est tout particulièrement utilisée comme intermédiaire clé entrant dans la synthèse de la D,L-méthionine et de son dérivé hydroxy-analogue, l'acide 2-hydroxy-4-méthylthiobutanoïque (HMTBA). Ces additifs alimentaires sont massivement employés car ils entrent dans la composition de compléments alimentaires indispensables à la croissance des animaux (volailles, porcs, ruminants, poissons, ...). Dans un certain nombre de cas, il peut être profitable de pouvoir augmenter, voire d'assurer les capacités de production des unités industrielles existantes en diversifiant la matière première engagée. Il apparaît donc tout particulièrement intéressant de pouvoir augmenter la productivité en acroléine, tout en réduisant la dépendance vis-à-vis de cette ressource issue du pétrole qu'est le propylène.

On connait des procédés de conversion du glycérol en acroléine, par déshydratation catalytique, selon la réaction :

$$HO\text{-}CH_2\text{-}CH(OH)\text{-}CH_2\text{-}OH \rightarrow CH_2\text{=}CH\text{-}CHO + 2H_2O$$

Le glycérol (appelé aussi glycérine) est depuis longtemps connu comme une source d'acroléine (transformation thermique), c'est un produit que l'on trouve largement dans la nature, sous forme d'esters (triglycérides), en particulier dans toutes les huiles et graisses animales ou végétales, ce qui en fait un réactif de départ disponible en quantité et en cela utilisable industriellement. Il est effectivement connu que le glycérol se décompose pour donner de l'acroléine lorsqu'il est porté à des températures supérieures à 280°C. Cette réaction faiblement sélective s'accompagne de la formation de nombreux sous-produits dont l'acétaldéhyde, l'hydroxyacétone, en plus des produits d'oxydation totale CO, $CO_2$. Il est donc indispensable de contrôler la réaction de transformation du glycérol en acroléine pour éviter un gaspillage inutile de cette ressource et s'affranchir d'une séparation postérieure énergétiquement coûteuse avec un procédé de purification de l'acroléine complexe. Par ailleurs, ces impuretés, principalement les dérivés aromatiques, sont souvent à l'origine de formation de coke à la surface du catalyseur qui empoisonne ce dernier au cours du temps; il est souvent nécessaire de régénérer le catalyseur de façon à retrouver une activité catalytique satisfaisante.

De nombreux chercheurs universitaires et industriels se sont penchés sur cette réaction. Il a notamment été envisagé d'utiliser de l'eau supercritique comme milieu réactionnel. L'utilisation de solvant supercritique à l'échelle industrielle demeure difficile pour un procédé continu en raison d'infrastructures particulièrement lourdes qui exigent des autoclaves fonctionnant sous très haute pression. En revanche, la mise en place d'un procédé continu ou discontinu devient envisageable si un système catalytique performant, sélectif et résistant est identifié.

Devant l'intérêt grandissant de cette alternative chimique, la littérature fait état d'un grand nombre d'études relatives à l'utilisation des systèmes catalytiques basés sur des hétéropolyacides phospho- ou silico-tungstiques supportés, des oxydes mixtes et des zéolithes, utilisables pour des procédés continus ou discontinus en phase liquide ou gazeuse.

Ainsi, les documents WO-A-2006087083 et WO-A-2006087084 décrivent un procédé de déshydratation catalytique de glycérol en acroléine en phase gazeuse, en présence d'oxygène moléculaire et d'un catalyseur fortement acide choisi parmi des zéolites, le Nafion®, des oxydes de métaux choisis parmi l'aluminium, le zirconium, le titane, le niobium, le tantale, le silicium, imprégnés par des fonctions acides sous forme de groupements sulfates, borates, tungstates, silicates et phosphates.

Le document WO-A-2007132926 divulgue un procédé de conversion du glycérol en acroléine en présence d'un catalyseur choisi parmi des métallosilicates cristallins acides tels que des zéolithes de type structural MFI ou BEA, comprenant du silicium et un élément de préférence choisi parmi Al, Fe et Ga.

FR2920767A1 décrit un procédé d'obtention d'acroléine ou d'acide acrylique à partir de glycérol, notamment issu d'EMVH. Le glycérol en solution aqueuse est vaporisé et mis au contact en phase gazeuse avec un catalyseur solide de déshydratation ou d'oxydéshydratation, en lit fluidisé. Le catalyseur employé est choisi parmi tout catalyseur adapté, notamment zéolites, composites Nafion®, alumines chlorées, acides et sels d'acides phosphotungstiques et/ou silicotungstiques et des oxydes métalliques imprégnés de fonctions acides. Un catalyseur de type $W/ZrO_2$-Si est notamment illustré. Ce catalyseur consiste en de l'oxyde de tungstène et de l'oxyde de zirconium, qui est ensuite enrobé de silice.

De même, US2008/214384A1 qui se rapporte à la même réaction, met en oeuvre un catalyseur acide, à base de tungstène, par exemple un catalyseur consistant en de la montmorillonite, un oxyde de tungstène et un oxyde de zirconium. Ce document décrit un procédé de régénération de ce catalyseur après qu'il ait servi dans la réaction de déshydratation du glycérol en acroléine où il a perdu de l'activité et/ou de la sélectivité. Cette régénération est réalisée par exposition dudit catalyseur seul, c'est-à-dire en l'absence de toute entité impliquée dans la déshydratation, à une atmosphère oxydante ou réductrice.

Ces catalyseurs présentent l'inconvénient de perdre rapidement leur réactivité et/ou leur sélectivité. Leur processus de régénération est généralement long et ne peut souvent pas être réalisé in situ. A titre d'illustration, le catalyseur à base de montmorillonite, d'oxyde de tungstène et d'oxyde de zirconium illustré dans US2008/214384A1 présente le comportement suivant :

- au bout de 5 heures sous mélange réactionnel, la réactivité du catalyseur est fortement diminuée ;
- le catalyseur ne peut pas être régénéré in situ, il est effectivement régénéré par un flux d'air à l'exclusion de toute autre entité ;
- sa régénération est longue, de l'ordre de 5 heures.

L'invention vise à lever les problèmes rencontrés avec les catalyseurs classiquement utilisés dans la réaction de déshydratation du glycérol en acroléine.

L'objet de la présente invention réside dans la mise en oeuvre de catalyseurs robustes, actifs, sélectifs et régénérables, permettant de produire de l'acroléine directement à partir de glycérol ou de glycérine, notamment issus de la biomasse.

Cette alternative permet ainsi de disposer d'un procédé compétitif de synthèse d'acroléine non dépendant de la ressource pétrolière propylène à partir d'une autre matière première renouvelable.

Cette possibilité est particulièrement avantageuse pour synthétiser la méthionine ou ses analogues, comme son hydroxy-analogue (HMTBA), directement à partir de la biomasse.

Ainsi, l'invention se rapporte en outre à une application de cette réaction à la synthèse de l'aldéhyde-3-(méthylthio)propionique (MMP), du 2-hydroxy-4-méthylthiobutyronitrile (HMTBN), de la méthionine et ses analogues tels que l'acide 2-hydroxy-4méthylthiobutanoïque (HMTBA), les esters du HMTBA comme l'ester isopropylique, l'acide 2-oxo-4-méthylthiobutanoïque, à partir d'acroléine.

La méthionine, le HMTBA et les esters de celui-ci et analogues, sont utilisés en nutrition animale et, dans leurs procédés industriels de synthèse, l'acroléine est généralement obtenue par oxydation du propylène et/ou du propane. L'oxydation du propylène en acroléine par l'air en présence d'eau est partielle, et le produit brut résultant, à base d'acroléine, contient aussi du propylène et du propane n'ayant pas réagi, de l'eau et des sous-produits de la réaction d'oxydation, notamment des acides, aldéhydes et alcools.

Par rapport aux procédés connus, on apporte, selon l'invention décrite, un procédé de préparation d'acroléine à partir de glycérol ou de glycérine, par déshydratation catalytique du glycérol en présence d'un catalyseur qui, tout en permettant de convertir la totalité du glycérol de départ, à la fois peut être très facilement régénéré et possède une longue durée de vie. Les auteurs de l'invention ont découvert que ce catalyseur est à base d'oxyde de zirconium et que sa phase active consiste en au moins :

un oxyde de silicium, un oxyde de zirconium, un oxyde de métal M qui le tungstène et un oxyde d'un autre métal M qui est le titane.

Les oxydes précités sont constitutifs de la phase active des catalyseurs de l'invention, c'est-à-dire qu'ils contribuent directement aux propriétés catalytiques du catalyseur. A titre d'exemple, un oxyde métallique agissant sur la sélectivité du catalyseur et/ou sur sa texture (taille de pore, taille de cristallite, surface spécifique) est constitutif de la phase active ; au contraire, un oxyde métallique ne jouant que le rôle de liant entre les particules de la phase active, ne saurait être considéré comme constitutif de la phase active selon l'invention.

Ainsi, l'invention concerne un procédé d'obtention d'acroléine à partir de glycérol ou de glycérine, en présence d'un catalyseur tel que défini ci-dessus, et l'utilisation de ce catalyseur, pour convertir du glycérol ou de la glycérine en acroléine. Un catalyseur de l'invention permet une conversion contrôlée du glycérol ou de la glycérine en acroléine, c'est-à-dire ne favorisant pas la conversion jusqu'à l'acide acrylique. A cet effet, un catalyseur préféré de l'invention ne comprend pas, ou ne comprend pas en proportion majoritaire en poids par rapport à chacun des autres oxydes le constituant, d'oxyde de molybdène et/ou d'oxyde de cuivre.

C'est pourquoi l'invention concerne aussi l'utilisation d'au moins l'un quelconque des catalyseurs tels que définis précédemment pour convertir du glycérol ou de la glycérine en acroléine.

Le catalyseur peut être préparé de diverses façons (coprécipitation, synthèse hydrothermale...). Une procédure efficace a été décrite dans les brevets FR2907444A1 et FR2907445A1.

Le catalyseur défini précédemment peut en outre répondre aux caractéristiques préférentielles ci-dessous, considérées seules ou en combinaison :

- le rapport molaire Zr/somme des autres éléments constitutifs desdits catalyseurs, c'est-à-dire choisis parmi Si, Zr et M , varie de 0,5 à 200, plus avantageusement, il varie de 1 à 100.

Comme dit précédemment, le catalyseur de l'invention présente l'intérêt de pouvoir être régénéré facilement, et ceci sans que le rendement de la déshydratation, ni la sélectivité en acroléine ne soient affectés.

La réaction selon l'invention peut être mise en oeuvre en phase gazeuse ou en phase liquide, de préférence en phase gazeuse. Lorsque la réaction est menée en phase gazeuse, différentes technologies de procédé peuvent être utilisées, à savoir procédé en lit fixe, procédé en lit fluidisé ou procédé en lit fluidisé circulant. Dans les deux premiers procédés, en lit fixe ou en lit fluidisé, la régénération du catalyseur peut être séparée de la réaction catalytique. Elle peut par exemple se faire *ex situ* par les méthodes de régénération conventionnelles, comme la combustion sous air ou avec un mélange gazeux contenant de l'oxygène moléculaire. Selon le procédé de l'invention, la régénération peut se faire *in situ* car les températures et pressions auxquelles se fait la régénération sont voisines des conditions réactionnelles du procédé.

S'agissant du procédé en phase liquide, la réaction peut être réalisée dans un réacteur classique pour réaction en phase liquide sur un catalyseur solide, mais aussi dans un réacteur de type distillation catalytique en égard à la différence significative des points d'ébullition du glycérol (290°C) et de l'acroléine (53°C). On peut également raisonnablement envisager un procédé en phase liquide à une température relativement basse qui permet une distillation continue de l'acroléine produite, limitant ainsi les réactions consécutives de dégradation de l'acroléine. Les conditions expérimentales de la réaction en phase gazeuse sont de préférence une température comprise entre 250 et 400°C à une pression comprise entre 1 et 10 bars. En phase liquide, la réaction est opérée entre 150 et 350°C et à une pression pouvant aller de 3 à 70 bars.

Un autre avantage du procédé de l'invention réside dans la forme du glycérol ou glycérine de départ qui peut être sous forme pure ou partiellement purifiée ou en solution, notamment aqueuse. Avantageusement, on utilise une solution aqueuse de glycérol. En solution aqueuse, la concentration du glycérol est de préférence d'au moins 1%, au mieux elle varie de 10 à 50% en poids et de préférence entre 15 et 30% en poids dans le réacteur. La concentration en glycérol ne doit pas être trop élevée dans le but d'éviter les réactions parasites qui grèvent le rendement en acroléine, comme la formation des éthers de glycérol ou des réactions d'acétalisation entre l'acroléine produite et le glycérol non converti. Par ailleurs, la solution de glycérol ne doit pas être trop diluée, en raison d'un coût énergétique rédhibitoire induit par l'évaporation du glycérol. Dans tous les cas, il est aisé d'ajuster la concentration de la solution de glycérol en recyclant partiellement ou totalement l'eau produite par la réaction considérée. L'optimisation énergétique aux bornes de la synthèse tend à récupérer la chaleur en sortie de réaction pour vaporiser le flux de glycérol alimenté au réacteur.

Un autre objet de l'invention est un procédé de fabrication de l'aldéhyde-3-(méthylthio)propionique (MMP), du 2-hydroxy-4-méthylthiobutyronitrile (HMTBN), de la méthionine, de l'acide 2-hydroxy-4-méthylthiobutanoïque (HMTBA), des esters de ce dernier, notamment l'ester isopropylique, et l'acide 2-oxo-4-méthylthiobutanoïque (KMB) à partir d'acroléine, qui comprend l'étape de déshydratation du glycérol ou glycérine en acroléine selon l'invention. Comparativement au procédé conventionnel de fabrication de l'acroléine par l'oxydation ménagée du propylène, l'acroléine produite selon le procédé susmentionné peut contenir des impuretés différentes du procédé traditionnel, tant sous l'angle de leur quantité que de leur nature. Selon l'utilisation envisagée, synthèse de l'acide acrylique ou de la méthionine ou son hydroxyanalogue, il pourra être envisagé de purifier l'acroléine selon les techniques connues de l'homme de l'art.

Ainsi, une fois l'acroléine directement obtenue selon l'invention ou après purification, elle est mise en réaction avec du méthylmercaptan (MSH) pour produire l'aldéhyde-3-(méthylthio)propionique (ou MMP). Dans une étape suivante, le MMP est mis en contact avec de l'acide cyanhydrique pour produire le 2-hydroxy-4-(méthylthio)butyronitrile (HMTBN). Après synthèse du HMTBN, diverses étapes réactionnelles conduisent à la méthionine, son hydroxyanalogue (HMTBA), les esters de ce dernier, ou son oxoanalogue (KMB). Toutes ces étapes à compter de la synthèse de l'acroléine sont bien connues de l'homme du métier.

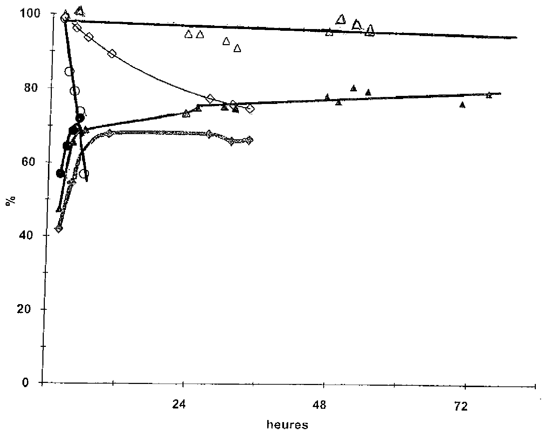

La présente invention est maintenant décrite plus en détail et illustrée avec les exemples et la figure ci-après sans toutefois en limiter la portée.

La Figure présente l'évolution de la conversion en glycérol et de la sélectivité en acroléine correspondante au cours du temps, sur le catalyseur E décrit dans l'exemple 4; le E est un catalyseur de l'invention, les catalyseurs F et G sont des catalyseurs de l'art antérieur. Le temps indiqué pour chaque point est celui de la fin d'un prélèvement correspondant à un piégeage pendant une heure. Les conditions réactionnelles et les méthodes de calcul utilisées par la conversion et la sélectivité en acroléine sont décrites plus loin.

Cette figure se lit à l'appui de la légende suivante:

- conversion en glycérol sur catalyseur E (△), F (◇) ou G(○)
- sélectivité en acroléine sur catalyseur E (▲), F (♦) ou G (●)

Les conditions réactionnelles et les méthodes de calcul de la conversion et de la sélectivité en acroléine sont décrites ci-après.

La réaction de déshydratation du glycérol est conduite sur les catalyseurs indiqués, à pression atmosphérique, dans un réacteur droit à lit fixe de diamètre 18mm. Le réacteur est placé dans un four qui permet de maintenir le catalyseur à la température de réaction qui est de 300°C. Le volume de catalyseur chargé dans le réacteur est de 4,5 mL, ce qui donne une hauteur de lit d'environ 1,8 cm. Le réacteur est alimenté avec un débit de 3,77 g/h de solution aqueuse à 20% en poids de glycérol. La solution aqueuse de glycérol est vaporisée grâce à un évaporateur C.E.M (Controlled Evaporator Mixer) Bronkhorst® en présence d'un débit d'azote de 75 mL/min. La proportion relative molaire glycérol/eau/azote est de 2,3/ 46,3/ 51,4. Le temps de contact calculé est de l'ordre de 1,9 s soit une GHSV de 1930 h$^{-1}$. Le temps de contact est défini comme suit :

$$\text{Temps de contact} = \text{Volume catalyseur} \times P_{atm} / (\text{débit molaire total} \times \text{Température} \times R)$$

avec $P_{atm}$=101325 Pa, Température =25°C et le débit molaire total = débit molaire du glycérol + débit molaire de l'eau + débit molaire du gaz inerte

Après réaction, les produits sont condensés. Deux systèmes de condensations ont été utilisés. Les exemples 7 à 10 ont été obtenus avec un système de trois pièges montés en série. Le premier piège contient une masse connue d'eau et est réfrigéré par de la glace pilée. Les deux autres pièges contiennent de l'éthanol et sont refroidis par un cryostat à -25°C. La durée de piégeage est d'une heure et le débit d'alimentation n'est pas interrompu pendant les changements de pièges.

Les produits formés sont analysés par chromatographie, deux analyses sont réalisées pour chaque prélèvement :

- Les principaux produits de la réaction sont analysés par chromatographie gazeuse sur une colonne capillaire (Nukol, 30m x 0,53 mm) avec un chromatographe Shimadzu 2014 muni d'un détecteur FID. Les produits quantifiés lors de cette analyse sont : l'acroléine, l'acétaldéhyde, l'acétone, le propionaldéhyde, l'hydroxypropanone, l'acide acétique, l'alcool allylique et le phénol ;
- Le glycérol restant est quantifié par chromatographie gazeuse avec un chromatographe Helwett Packard équipé d'un détecteur FID et d'une colonne capillaire (Carbowax ou ZBwax, 30 m x 0,32 mm).

La conversion en glycérol, la sélectivité en acroléine et les rendements en différents produits sont définis comme suit :

$$\text{Conversion en glycérol (\%)} = 100 \times (1-\text{nombre de moles de glycérol restantes/nombre de moles de glycérol introduites})$$

$$\text{Sélectivité en acroléine (\%)} = 100 \times (\text{nombre de moles d'acroléine produites /nombre de moles de glycérol ayant réagi})$$

$$\text{Rendement en X (\%)} = K \times 100 \times \text{nombre de moles de X produites / nombre de moles de glycérol introduites}$$

Avec K= 1 si X est l'acroléine, l'acétone, l'hydroxypropanone, le propanal ou l'alcool allylique; K=2/3 si X= acétaldéhyde ou acide acétique et K=2 si X= phénol.

Exemple 1 : préparation et caractérisation du catalyseur A (hors invention)

Ce catalyseur est de type zircone tungstée dopée à la silice. La préparation de ce solide comporte trois étapes. La première étape est la synthèse de l'hydroxyde de zirconium hydraté par co-précipitation d'une solution d'oxonitrate de zirconium $ZrO(NO_3)_2.xH_2O$ (Aldrich, 99%) et d'une solution d'ammoniaque à 28% à pH=8,8. La deuxième étape consiste à stabiliser l'hydroxyde de zirconium hydraté par des espèces siliciques selon la procédure décrite par Nahas et al. (Journal of Catalysis 247 (2007), p51-60). L'hydroxyde de zirconium hydraté est placé dans un ballon en verre contenant une solution ammoniacale dont le pH est ajusté à 11. Le mélange est chauffé à reflux pendant 72h puis filtré et lavé à l'eau permutée. La dernière étape est l'échange entre l'acide tungstique $H_2WO_4$ (Aldrich, 99%) dissous dans du peroxyde d'hydrogène et l'hydroxyde de zirconium. L'acide tungstique est dissous dans une solution de peroxyde d'hydrogène à 35% à 60°C. La concentration de la solution en acide tungstique est de 0,04M. La solution d'acide tungstique est ensuite refroidie à température ambiante, et l'hydroxyde de zirconium dopé à la silice est ajouté lentement. Le solide obtenu est filtré puis calciné sous air à 650°C. Sa surface spécifique est de 40m$^2$/g. Les teneurs en niobium, silicium et zirconium du solide ont été déterminées par ICP-OES. La composition molaire W/Si/Zr de ce catalyseur est 4,7/1,4/93,9.

Exemple 2 : Préparation et caractérisation du catalyseur B et C (hors invention)

Deux catalyseurs de type zircone tungstée dopée à la silice, sont préparés. Les catalyseurs sont préparés avec le même protocole que celui du catalyseur A mais, lors de la deuxième étape, le mélange est chauffé à reflux seulement 24h puis filtré et lavé à l'eau permutée. La concentration de la solution en acide tungstique est de 0,04M pour le catalyseur B et de 0,1M pour le catalyseur C. Leurs surfaces spécifiques sont respectivement de 92 et 82 $m^2$/g. Les teneurs en tungstène, silicium et zirconium des solides ont été déterminées par ICP-OES. Leurs compositions molaires $ZrO_2$/$SiO_2$/$WO_3$ sont de 90,6/0,7/8,7 pour B et 87,3/0,6/12,1 pour C.

Exemple 3 : Préparation et caractérisation du catalyseur D (ne relève pas de l'invention)

Le catalyseur D est de type zircone tungstée. Il est préparé avec le même protocole que celui du catalyseur B mais sans la deuxième étape, c'est-à-dire sans ajout de silice. La surface spécifique est de 92$m^2$/g. Les teneurs en tungstène et zirconium du solide ont été déterminées par ICP-OES. La composition molaire $ZrO_2$/$WO_3$ de ce catalyseur est 92,5/7,5.

Exemple 4 : préparation et caractérisation du catalyseur E (selon l'invention)

Le catalyseur ZrTiSiW selon l'invention a été préparé par Rhodia selon la méthode décrite dans le brevet FR2907445A1. La surface spécifique de ce catalyseur a été mesurée par la méthode BET (Brunauer Emmet et Teller) à -196°C sur un appareil Micromeritics ASAP 2020. Les solides sont préalablement désorbés à 300°C pendant 3 heures sous un vide de $5x10^{-5}$ mbar, est de 105$m^2$/g. La composition en poids d'oxydes de ce catalyseur est 54% de $ZrO_2$, 35% de $TiO_2$, 7,5% de $SiO_2$ et 3,5% de $WO_3$.

Exemple 5 : préparation et caractérisation du catalyseur F (comparatif de l'art antérieur)

Le catalyseur F est une zircone tungstée (89,5% $ZrO_2$-10,5% $WO_3$) synthétisé par Daiichi Kigenso (référence fournisseur : Z-1104). La surface spécifique de ce catalyseur est de 77 $m^2$/g.

Exemple 6 : préparation et caractérisation du catalyseur G (comparatif de l'art antérieur)

Le catalyseur G est une zéolithe H-ZSM-5 fournie par Zeochem (ZEOcat PZ-2/5OH). La surface spécifique de ce catalyseur est de 406 $m^2$/g.

Exemple 7: Déshydratation catalytique du glycérol en acroléine : évaluation des catalyseurs E, F et G

Le tableau 1 donne les performances obtenues avec les catalyseurs E, F et G à 6 heures de réaction.

Tableau 1 :

|  | E (invention) | F (comparatif) | G (comparatif) |
|---|---|---|---|
| Conversion du glycérol | 100 | 94 | 57 |
| Sélectivité en acroléine | 69 | 64 | 65 |
| Rendement en acroléine | 69 | 60 | 37 |
| Rendement en acétaldéhyde | 6,5 | 3,9 | 0,6 |
| Rendement en propionaldéhyde | 5,4 | 2,8 | 1,6 |
| Rendement en acétone | 2,7 | 1,6 | 0,0 |
| Rendement en alcool allylique | 0,5 | 0,5 | 0,2 |
| Rendement en hydroxypropanone | 3,1 | 6,1 | 3,0 |
| Rendement en phénol | 0,8 | 0,3 | - |

Ce tableau montre qu'à volume égal de catalyseur, seul le catalyseur E (selon l'invention) permet une conversion totale du glycérol. De plus, le catalyseur de l'invention permet d'obtenir une meilleure sélectivité en acroléine, déjà visible à 6 h et qui se confirme à 50 h, avec un rendement en acroléine de 80% pour le catalyseur E.
Le catalyseur E est donc plus actif et plus sélectif que les catalyseurs de l'art antérieur.

Exemple 8 : Déshydratation catalytique du glycérol en acroléine : Evolution au cours du temps des performances des catalyseurs E, F et G

L'évolution des performances des catalyseurs E, F et G au cours du temps, obtenues dans les mêmes conditions que dans l'exemple 7 est présentée sur la figure.

Le catalyseur E de l'invention maintient une sélectivité constante en acroléine et une conversion en glycérol élevée sur plusieurs jours contrairement aux catalyseurs F et G de l'art antérieur qui se désactivent fortement en moins de 24h.

Le catalyseur E de l'invention est donc plus actif, plus sélectif en acroléine mais aussi plus stable dans le temps que les meilleurs catalyseurs revendiqués dans l'art antérieur.

Exemple 9: Déshydratation catalytique du glycérol en acroléine : évaluation du catalyseur A (hors l'invention)

Le tableau 2 donne les performances du catalyseur A.

Tableau 2 :

| heures de fin du prélèvement | 4 | 23 | 42 |
|---|---|---|---|
| Conversion du glycérol | 98 | 96 | 87 |
| Sélectivité en acroléine | 68 | 80 | 83 |
| Rendement en acroléine | 67 | 77 | 72 |
| Rendement en acétaldéhyde | 4,2 | 3,5 | 2,4 |
| Rendement en propionaldéhyde | 3,1 | 2,4 | 1,6 |
| Rendement en acétone | 1,2 | 1,3 | 0,9 |
| Rendement en alcool allylique | 0,7 | 0,9 | 0,6 |
| Rendement en hydroxypropanone | 5,2 | 10,9 | 9,7 |
| Rendement en phénol | 0,8 | 0,2 | - |

Exemple 10: Déshydratation catalytique du glycérol en acroléine : évaluation des catalyseurs C, B et D (hors invention)

Le tableau 3 donne les performances des catalyseurs B, C et D obtenues. L'effet du silicium ajouté en tant que dopant à faible teneur apparaît clairement à travers la comparaison des catalyseurs B et D. La comparaison des catalyseurs B et C montre que des performances aussi bonnes ou meilleures peuvent être obtenues avec plus de tungstène.

Tableau 3

| | catalyseur D | | catalyseur B | | | | catalyseur C | | | |
|---|---|---|---|---|---|---|---|---|---|---|
| Heure de fin de prélèvement | 8 | 29 | 8 | 30 | 75 | 97 | 6 | 29 | 71 | 145 |
| Conversion du glycérol | 100 | 71 | 100 | 100 | 96 | 94 | 100 | 99 | 95 | 77 |
| Rendement en acroléine | 61 | 50,2 | 63,2 | 75,6 | 75,3 | 72,6 | 65,3 | 74,3 | 77,7 | 56,9 |
| Rendement en acétaldéhyde | 4,9 | 2,5 | 7,4 | 6,8 | 5,1 | 4,3 | 7,3 | 6,0 | 4,0 | 1,9 |
| Rendement en propionaldéhyde | 5,7 | 4,0 | 5,3 | 4,7 | 3,7 | 3,4 | 4,6 | 3,7 | 3,0 | 1,4 |
| Rendement en acétone | 1,9 | 0,7 | 2,5 | 2,6 | 2,3 | 2,0 | 2,7 | 3,0 | 2,2 | 1,0 |
| Rendement en alcool allylique | 0,4 | 0,9 | - | 0,5 | 0,8 | 1,1 | 0,6 | 0,2 | 0,5 | 0,8 |
| Rendement en hydroxypropanone | 3,6 | 8,6 | 0,9 | 4,9 | 7,9 | 9,0 | 4,5 | 4,1 | 8,2 | 9,1 |
| Rendement en phénol (%) | 1,1 | 0,1 | 1,8 | 0,8 | 0,3 | 0,2 | 1,7 | 0,4 | 0,1 | - |

Masse de catalyseur, utilisée dans les conditions standards : 6,96 g.

**Revendications**

1. Procédé de préparation d'acroléine à partir de glycérol, ou de glycérine, **caractérisé en ce qu'**on réalise la déshydratation du glycérol ou glycérine en présence d'un catalyseur à base d'oxyde de zirconium et dont la phase active consiste en au moins un oxyde de silicium, un oxyde de zirconium, un oxyde de métal M qui est le tungstène et un oxyde d'un autre métal M qui est le titane.

2. Procédé selon la revendication 1, **caractérisé en ce que** le rapport molaire Zr/somme des éléments Si, Ti et M, différents de Zr varie de 0,5 à 200.

3. Procédé la revendication 2, **caractérisé en ce que** ledit rapport molaire varie de 1 à 100.

4. Procédé selon l'une quelconque des revendications 1 à 3, **caractérisé en ce que** le glycérol est en solution aqueuse, en une concentration d'au moins 1% en poids.

5. Procédé selon la revendication 4, **caractérisé en ce que** la concentration de la solution aqueuse en glycérol varie de 10 à 50% en poids.

6. Procédé selon l'une quelconque des revendications 1 à 5, **caractérisé en ce que** le catalyseur est régénéré.

7. Procédé de fabrication de l'aldéhyde-3-(méthylthio)propionique MMP, du 2-hydroxy-4-méthylthiobutyronitrile HMTBN, de la méthionine, de l'acide 2-hydroxy-4-méthylthiobutanoïque HMTBA, des esters de ce derniers, ou du 2-oxo-4-méthylthiobutanoïque KMB, à partir d'acroléine, **caractérisé en ce que** l'acroléine est obtenue par un procédé selon l'une quelconque des revendications 1 à 6.

8. Procédé selon l'une des revendications 1 à 7, **caractérisé en ce que** la réaction de déshydratation est réalisée en phase gazeuse.

9. Procédé selon la revendication 8, **caractérisé en ce que** la réaction de déshydratation est réalisée dans un réacteur à lit fixe, à lit fluidisé ou à lit fluidisé circulant.

10. Procédé selon l'une des revendications 1 à 7, **caractérisé en ce que** la réaction de déshydratation est réalisée en phase liquide.

11. Utilisation d'un catalyseur tel que défini dans la revendication 1 ou 2 et éventuellement 6, pour convertir du glycérol ou de la glycérine en acroléine.


**Patentansprüche**

1. Verfahren zur Herstellung von Acrolein, ausgehend von Glycerol oder Glycerin, **dadurch gekennzeichnet, dass** die Dehydrierung von Glycerol oder Glycerin durchgeführt wird in Anwesenheit eines Katalysators, ausgehend von Zirkoniumoxid, und wobei die aktive Phase mindestens aus einem Siliziumoxid, einem Zirkoniumoxid, einem Metalloxid M, wobei es sich um Wolfram handelt, und einem Oxid eines anderen Metalls M, wobei es sich um Titan handelt, besteht.

2. Verfahren nach Anspruch 1, **dadurch gekennzeichnet, dass** das Molverhältnis Zr/Summe der Elemente Si, Ti und M, verschieden von Zr, von 0,5 bis 200 variiert.

3. Verfahren nach Anspruch 2, **dadurch gekennzeichnet, dass** das Molverhältnis von 1 bis 100 variiert.

4. Verfahren nach einem der Ansprüche 1 bis 3, **dadurch gekennzeichnet, dass** das Glycerol in wässriger Lösung in einer Konzentration von mindestens 1 Gew% vorliegt.

5. Verfahren nach Anspruch 4, **dadurch gekennzeichnet, dass** die Konzentration der wässrigen Lösung in Glycerol von 10 bis 50 Gew% variiert.

6. Verfahren nach einem der Ansprüche 1 bis 5, **dadurch gekennzeichnet, dass** der Katalysator regeneriert ist.

**7.** Verfahren zur Herstellung von 3-(Methylthio)propionaldehyd MMP, 2-Hydroxy-4-methylthiobutyronitril HMTBN, Methionin, 2-Hydroxy-4-methylthiobutansäure HMTBA, Estern dieser letzteren oder 2-Oxo-4-methylthiobutan KMB, ausgehend von Acrolein, **dadurch gekennzeichnet, dass** das Acrolein erhalten wird durch ein Verfahren nach einem der Ansprüche 1 bis 6.

**8.** Verfahren nach einem der Ansprüche 1 bis 7, **dadurch gekennzeichnet, dass** die Dehydrierungsreaktion in der gasförmigen Phase durchgeführt wird.

**9.** Verfahren nach Anspruch 8, **dadurch gekennzeichnet, dass** die Dehydrierungsreaktion in einem Reaktor mit festem Bett, mit verflüssigtem Bett oder mit zirkulierendem verflüssigtem Bett durchgeführt wird.

**10.** Verfahren nach einem der Ansprüche 1 bis 7, **dadurch gekennzeichnet, dass** die Dehydrierungsreaktion in der flüssigen Phase durchgeführt wird.

**11.** Verwendung eines Katalysators, wie in Anspruch 1 oder 2 und eventuell 6 definiert, um Glycerol oder Glycerin in Acrolein umzuwandeln.

**Claims**

**1.** A method for preparing acrolein from glycerol or from glycerin, **characterized in that** the dehydration of glycerol or glycerin is carried out in the presence of a zirconium oxide-based catalyst and whose active phase consists of at least one silicon oxide, one zirconium oxide, one metal oxide M which is tungsten and one oxide of another metal M which is titanium.

**2.** The method according to claim 1, **characterized in that** the molar ratio Zr/sum of the elements Si, Ti and M, different from Zr ranges from 0.5 to 200.

**3.** The method according to claim 2, **characterized in that** said molar ratio ranges from 1 to 100,

**4.** The method according to any one of claims 1 to 3, **characterized in that** the glycerol is in aqueous solution, at a concentration of at least 1% by weight.

**5.** The method according to claim 4, **characterized in that** the concentration of the aqueous glycerol solution ranges from 10 to 50% by weight.

**6.** The method according to any one of claims 1 to 5, **characterized in that** the catalyst is regenerated.

**7.** The method for manufacturing 3-(methylthio)propionaldehyde MMP, 2-hydroxy-4-methylthiobutyronitrile HMTBN, methionine, 2-hydroxy-4-methylthiobutanoic acid HMTBA, esters thereof, or 2-oxo-4-methylthiobutanoic acid KMB, from acrolein, **characterized in that** the acrolein is obtained by a method according to any one of claims 1 to 6.

**8.** The method according to any of claims 1 to 7, **characterized in that** the dehydration reaction is carried out in the gas phase.

**9.** The method according to claim 8, **characterized in that** the dehydration reaction is carried out in a fixed bed, fluidized bed or circulating fluidized bed reactor.

**10.** The method according to any of claims 1 to 7, characterized the dehydration reaction is carried out in the liquid phase.

**11.** A use of a catalyst as defined in claim 1 or 2 and optionally 6, to convert glycerol or glycerin into acrolein.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2006087083 A
- WO 2006087084 A
- WO 2007132926 A
- FR 2920767 A1
- US 2008214384 A1
- FR 2907444 A1
- FR 2907445 A1

**Littérature non-brevet citée dans la description**

- **M. PAGLIARO et al.** *Angew. Chem. Int. Ed.,* 2007, vol. 46, 4434-4440
- **M. PAGLIARO ; M. ROSSI.** The Future of Glycerol. RSC Publishing, 2008
- **NAHAS et al.** *Journal of Catalysis,* 2007, vol. 247, 51-60